# FASCICULE DE BREVET EUROPEEN

(11) **EP 3 752 230 B1**
(45) Date de publication et mention de la délivrance du brevet: **03.04.2024**
(21) Numéro de dépôt: 19710738.6
(22) Date de dépôt: 12.02.2019
(51) Int. Cl.: A61B 5/15, A61M 5/32, A61M 5/31, A61M 25/06, A61B 5/153

(54) **TETE D'INJECTION D'UNE SUBSTANCE FLUIDE PERMETTANT LE CONTROLE DU SITE D'INJECTION AVANT INJECTION DE LA SUBSTANCE FLUIDE ET SERINGUE COMPRENANT UNE TELLE TETE**
INJEKTIONSKOPF ZUR INJEKTION EINER FLÜSSIGEN SUBSTANZ UND ZUR ÜBERWACHUNG DER INJEKTIONSSTELLE VOR INJEKTION DER FLÜSSIGEN SUBSTANZ UND SPRITZE MIT EINEM SOLCHEN KOPF
INJECTION HEAD FOR INJECTING A FLUID SUBSTANCE AND PERMITTING MONITORING OF THE INJECTION SITE BEFORE INJECTION OF THE FLUID SUBSTANCE, AND SYRINGE COMPRISING SUCH A HEAD

(30) Priorité: 12.02.2018 FR 1851149
(43) Date de publication de la demande: 23.12.2020
(73) Titulaire: Adda, Jean-Marc, 92100 Boulogne-Billancourt (FR)
(72) Inventeur: CORBIN, Jean-Yves, 14480 LE FRESNE CAMILLY (FR); D'ESTAIS, Mathias, 14000 CAEN (FR); VAUPRES, Maxime, 14540 GRENTHEVILLE (FR); ADDA, Jean-Marc, 92100 BOULOGNE-BILLANCOURT (FR)
(74) Mandataire: IP Trust
(86) Numéro de dépôt international: PCT/FR2019/050304
(87) Numéro de publication internationale: WO 2019/155177

(56) Documents cités:
- EP-A1- 0 998 953
- EP-A1- 1 317 938
- WO-A1-2015/082566
- US-A- 6 096 005
- US-A1- 2015 025 466

## Description

### DOMAINE TECHNIQUE DE L'INVENTION

L'invention concerne le domaine de l'injection sous cutanée de substances fluides (produit de comblement, dérivé sanguin, produit de sclérose sans que cette liste soit restrictive).

L'invention concerne plus particulièrement une tête d'injection et une seringue équipée d'une telle tête d'injection, pour l'injection d'une substance fluide dans un site d'injection corporel en passant la barrière cutanée, ladite tête permettant, préalablement à l'injection, de contrôler la présence d'un liquide en provenance du site d'injection et de caractériser ledit liquide.

La tête d'injection selon l'invention est destinée notamment, mais non exclusivement, à l'injection sous cutanée d'une substance fluide exogène, en particulier d'un acide hyaluronique, à des fins esthétiques comme le comblement d'irrégularités en creux de la peau comme par exemple des rides.

La tête d'injection selon l'invention peut être prévue également pour l'injection d'une substance fluide endogène, préalablement prélevée ou ponctionnée d'un corps, pour être réinjectée dans un site d'injection corporel après avoir été le cas échéant traitée.

### ETAT DE LA TECHNIQUE

Le recours à des gestes d'esthétiques médicales et chirurgicales par injection sous cutanée de fluides pour l'atténuation des rides (fluides de comblement) est en constante augmentation. De ce fait, le risque d'accidents par thrombose vasculaire augmente exponentiellement.

L'injection de fluides de comblement présente ainsi certains risques dont un majeur qui est l'injection du fluide dans un vaisseau sanguin. Dans la présente invention, on entend par vaisseau sanguin, le réseau artériel et le réseau veineux. Or l'injection d'un fluide de comblement dans un vaisseau sanguin peut provoquer une obstruction à la circulation sanguine (thrombose), celle-ci pouvant être plus ou moins grave selon la localisation du vaisseau touché.

Afin de limiter le risque d'injection du fluide de comblement dans un vaisseau sanguin, un protocole de contrôle de présence de sang par aspiration a été mis en place. Un exemple de seringue permettant l'aspiration de contrôle avant la réalisation de l'injection est proposé dans la demande de brevet FR2659858. La seringue décrite comprend un embout de seringue transparent pourvu d'une ouverture ovale, un manchon transparent disposé à l'intérieur de l'embout, ledit manchon étant réalisé en matière plastique souple transparent, et une embase d'aiguille transparente apte à être montée sur l'embout jusqu'à l'ouverture ovale. Le contrôle de la présence de sang et donc de l'éventuelle implantation de l'aiguille dans un vaisseau sanguin est réalisé de la manière suivante. Le manipulateur pince l'embase de l'aiguille entre le pouce et l'index de la main droite, puis avec le pouce de l'autre main, appuie sur le manchon au travers de l'ouverture ovale et le collabe. Il retire ensuite l'embout de l'embase de l'aiguille. Lors du retrait de l'embout de l'embase, l'apparition d'un volume de sang dans l'embout indique que l'aiguille est implantée dans un vaisseau sanguin. Dans ce cas, le manipulateur ne procède pas à l'injection du fluide. Au contraire, si aucune remontée de sang n'apparait dans l'embout, cela signifie que l'aiguille n'est pas implantée dans un vaisseau sanguin. Dans ce cas, le manipulateur peut procéder à l'injection du fluide de comblement. Pour ce faire, il met fin à la pression exercée sur le manchon, replace l'embout au fond de l'embase et procède à l'injection du fluide par poussée sur le piston de la seringue.

La seringue de la demande susmentionnée présente cependant un inconvénient majeur, en ce qu'elle n'est pas utilisable pour deux cycles d'injection successifs si l'on utilise un fluide d'injection visqueux. En effet, suite à un premier cycle d'injection, l'aiguille est pleine de fluide à injecter. Pour la rendre apte à effectuer le contrôle d'un nouveau site d'injection, il est nécessaire de libérer l'aiguille et l'embase transparente en déplaçant le fluide d'injection présent dans l'aiguille par aspiration vers la seringue. Or, la dépression étant réalisée en amont de l'aiguille, il peut arriver que la remontée du sang ne se fasse pas alors même que l'aiguille a pénétré un vaisseau sanguin. Cela peut s'expliquer du fait d'une aspiration insuffisante, mais peut aussi se constater sur une aspiration très forte mais inopérante du fait des frottements du fluide visqueux à injecter dans une aiguille de très faible diamètre.

Il faut noter que la viscosité des produits de comblement est importante et garante de la qualité de l'acte esthétique, et que ces dernières années cette viscosité a largement augmenté, rendant la seringue proposée dans ledit brevet inopérante dans la majeure partie des cas.

Par ailleurs, on note que cette conception nécessite une manipulation à deux mains rendant le geste peu aisé en particulier pour des personnes non formées.

D'autres dispositifs d'injection de substances fluides mettant en oeuvre un mécanisme de rétraction d'aiguille sont également connus. Citons par exemple les documents US6096005, EP0998953, EP1317938 et US2015/025466.

L'invention vise à remédier à ces problèmes en proposant une tête d'injection ainsi qu'une seringue comportant une telle tête permettant de contrôler rapidement, simplement et de manière précise le site dans lequel l'aiguille est implantée tout en permettant une injection de la substance fluide sans manipulation complémentaire en cas de contrôle de plusieurs sites avant injection et vise également à pouvoir utiliser la même seringue pour plusieurs sites d'injections.

### OBJET DE L'INVENTION

L'invention est définie par les caractéristiques de la revendication 1 et des revendications dépendantes.

A cet effet, et selon un premier aspect, l'invention propose une tête d'injection d'une substance fluide destinée à être injectée dans un site d'injection corporel en passant la barrière cutanée, ladite tête permettant, préalablement à l'injection, le contrôle de la présence d'une substance fluide en provenance du site d'injection et de sa caractérisation. L'invention est remarquable en ce qu'elle présente un corps de liaison destiné à être connecté à un réservoir dont la pression peut être contrôlée, ledit réservoir étant destiné à contenir la substance fluide à injecter, ledit corps de liaison étant prolongé par un tube creux, destiné à communiquer avec le débouché du réservoir, présentant une extrémité distale, et une aiguille présentant une extrémité distale pointue, ledit tube creux ou ledit corps de liaison présentant au moins une zone de contrôle et de caractérisation de la substance fluide en provenance du site d'injection, des moyens pour commander le déplacement relatif de ladite aiguille par rapport audit tube creux, entre une position de piquage dans laquelle l'extrémité pointue de l'aiguille, laquelle est gigogne dans le tube creux, dépasse l'extrémité distale du tube creux, pour former, avec ladite extrémité distale, un ensemble pénétrant, et une position de contrôle et de caractérisation de la substance fluide en provenance du site d'injection via la zone de contrôle et de caractérisation, l'extrémité distale pointue de l'aiguille étant en retrait de l'extrémité distale du tube creux dans ladite position.

Les moyens de commande comportent une navette montée mobile par rapport au corps de liaison pour placer l'aiguille en position de piquage ou en position de contrôle et de caractérisation d'un liquide en provenance du site d'injection.

Les moyens de commande comportent des moyens de rappel élastique de la navette pour placer l'aiguille en position de piquage ou en position de contrôle et de caractérisation d'un liquide en provenance du site d'injection.

Avantageusement, le corps de liaison comporte des moyens de guidage coopérant avec des moyens de guidage complémentaires ménagés sur la navette de sorte à guider la navette dans son parcours.

Avantageusement, la navette et le corps de liaison comporte des moyens de maintien coopérant de sorte à maintenir l'aiguille dans sa position de piquage ou sa position de contrôle et de caractérisation du liquide en provenance du site d'injection.

Avantageusement, l'aiguille est pleine ou creuse.

Selon l'invention, l'aiguille est solidaire de la navette tandis que le tube creux est solidaire du corps de liaison. L'aiguille est montée coulissante dans un canal de guidage traversant le corps de liaison. Le tube creux communique avec le débouché du réservoir via un canal d'injection traversant le corps de liaison et présentant une extrémité débouchante dans le canal de guidage. Il peut être prévu également que les moyens de commande sont arrangés pour commander le déplacement de l'aiguille dans le canal de guidage dans une position d'injection de la substance fluide dans laquelle l'aiguille est positionnée en amont de l'extrémité débouchante du canal d'injection. Préférentiellement, l'aiguille est pleine.

L'invention peut être également mise en oeuvre en connectant un système de délivrance d'actifs de type pousse-seringue ou pompe, délivrant de l'actif ou toute autre substance fluide à la demande via un tuyau, à la tête d'injection étant raccordée tuyau.

La tête d'injection selon l'invention, lorsque raccordée à une seringue ou tout autre dispositif de délivrance de substance fluide, permet de sécuriser l'acte d'injection pour éviter tout risque de nécrose par obturation d'une artère du patient. Elle permet également de confirmer le site d'injection afin de s'assurer de la bonne délivrance d'un produit au bon endroit.

L'invention concerne également une seringue équipée, en partie avant, d'une tête d'injection telle que décrite précédemment, ladite tête d'injection étant raccordée au réservoir de la seringue. Avantageusement, la tête d'injection et le réservoir de la seringue constituent deux pièces indépendantes, fixables l'une à l'autre.

Avantageusement, la tête d'injection est amovible.

Avantageusement, le réservoir est pré-rempli. Dans le cas de l'acide hyaluronique, la seringue présente un volume pré-rempli habituellement compris entre 0,5 à 2cc.

L'indépendance entre la tête d'injection et le réservoir de la seringue présente plusieurs avantages.

En premier lieu, cette indépendance permet de changer la tête d'injection en fonction des conditions d'intervention. Dans le cas de l'utilisation d'une seringue pré-remplie, le praticien choisit dans un premier temps la référence de produit actif et le volume qu'il souhaite appliquer en fonction de l'indication et de la zone à traiter. Il choisit ensuite la gauge et la longueur de l'aiguille qu'il va utiliser pour l'injection (par exemple une aiguille de 25 mm de 30 Gauge). Le choix de la seringue (actif et volume) et le choix de la tête d'injection (gauge et longueur) sont indépendants et sont faits par le praticien au moment d'opérer. L'indépendance de la tête d'injection avec le corps de la seringue permet donc de moduler selon les besoins et les conditions d'intervention tout en assurant les conditions de sécurité vis-à-vis des accidents de piqûres et des risques de contamination.

Cette indépendance permet aussi de conserver au cours d'une intervention, si nécessaire, la même tête d'injection et de changer de seringue. Elle permet également de changer de seringue alors que l'aiguille reste insérée dans la peau.

L'invention concerne également l'utilisation d'une tête d'injection d'une substance fluide en combinaison avec une seringue pour contrôler, préalablement à l'injection d'une substance fluide dans un site d'injection corporel dans lequel la seringue est implantée, la présence d'un vaisseau sanguin au niveau du site d'injection.

### BREVE DESCRIPTION DES FIGURES

D'autres objets et avantages de l'invention apparaîtront au cours de la description qui suit, faite en référence aux dessins annexés, dans lesquels :
- la figure 1 représente une vue schématique en coupe d'une seringue pourvue d'une tête d'injection selon un premier mode de réalisation, ladite tête d'injection étant en position de piquage ;
- la figure 2 représente une vue schématique en coupe de la seringue illustrée sur la figure 1, la tête d'injection étant en position de contrôle et de caractérisation d'un liquide en provenance du site d'injection dans lequel la seringue est plantée ;
- les figures 3a à 6b représentent le mode opératoire pour l'injection d'un fluide de comblement dans un site d'injection au moyen de la seringue illustrée sur la figure 1, incluant une étape de contrôle du site dans lequel l'aiguille de la seringue est plantée avant injection ;
- les figures 7 et 8 représentent respectivement deux variantes de réalisation de la tête d'injection dans la configuration aiguille pleine;
- la figure 9 représente une autre variante de réalisation de la tête d'injection, notamment de la zone de contrôle et de caractérisation du liquide en provenance du site d'injection ;
- la figure 10 représente une vue schématique en coupe d'une seringue pourvue d'une tête d'injection selon un autre mode de réalisation ne faisant pas partie de l'invention, ladite tête d'injection étant en position de piquage ;
- la figure 11 représente une vue schématique en coupe de la seringue illustrée sur la figure 10, la tête d'injection étant en position de contrôle et de caractérisation d'un liquide en provenance du site d'injection dans lequel la seringue est plantée;
- les figures 12a à 15b représentent le mode opératoire pour l'injection d'un fluide de comblement dans un site d'injection au moyen de la seringue illustrée sur la figure 10, incluant une étape de contrôle du site dans lequel l'aiguille de la seringue est plantée avant injection.

Pour plus de clarté, les éléments identiques ou similaires des différents modes de réalisation sont repérés par des signes de référence identiques sur l'ensemble des figures.

### DESCRIPTION DETAILLEE DES FIGURES

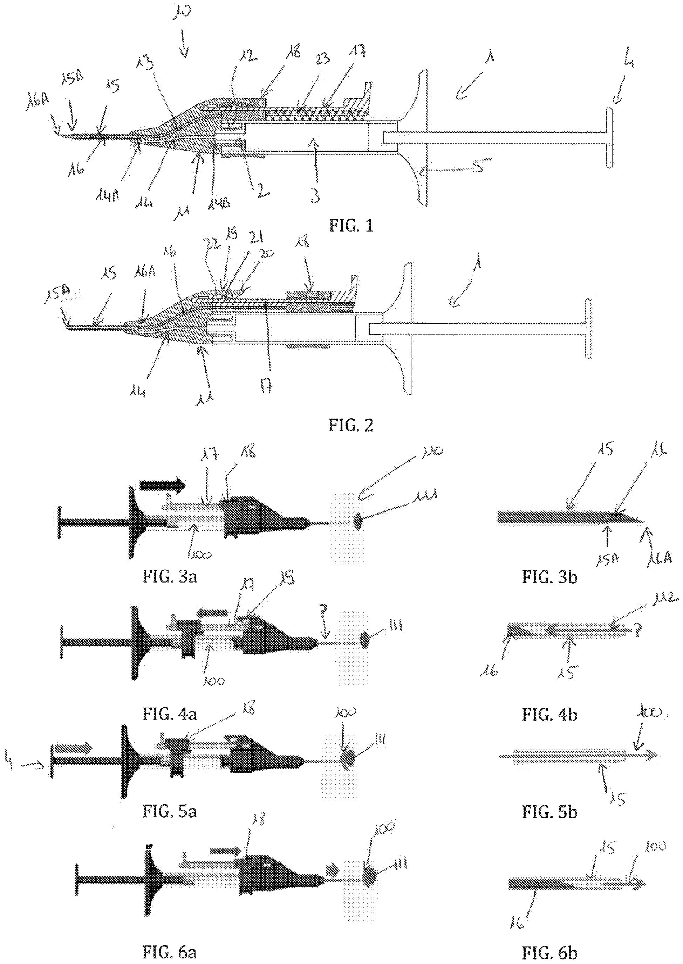

En relation avec les figures 1 à 6, il est décrit une tête d'injection 10 d'une substance fluide 100 destinée à être injectée dans un site d'injection corporel 110. Comme on le verra plus loin, la tête d'injection 10 permet, préalablement à l'injection de la substance fluide 100, de contrôler le site d'injection 110 en vérifiant la présence ou non d'une substance liquide en provenance du site d'injection 110 et en caractérisant ledit liquide. Dans le mode de réalisation décrit, la tête d'injection 10 équipe une seringue 1 destinée à injecter un fluide exogène, à savoir un fluide de comblement tel qu'un acide hyaluronique ou tout autre produit d'injection.

Dans le mode de réalisation décrit, la tête d'injection 10 comporte un corps de liaison 11 fixé sur la partie avant de la seringue 1. Par partie avant de la seringue, on entend la partie de la seringue portant l'embout 2 constituant le débouché du réservoir 3 de la seringue 1. Plus particulièrement, le corps de liaison 11 comporte, en partie arrière, un élément de couplage 12 constituant une connexion du type Luer Lock femelle apte à coopérer avec l'embout 2 de la seringue 1, l'embout 2 formant une connexion du type Luer Lock mâle. Il s'agit bien entendu d'un exemple de couplage, tout autre type de couplage pouvant être mis en oeuvre sans sortir du champ de l'invention, notamment Luer Slip.

Le corps de liaison 11 est traversé par deux canaux 13, 14 s'étendant de part en part des parties avant et arrière du corps de liaison 11.

L'un des canaux 13, qui définit un canal de guidage pour une aiguille avantageusement pleine comme on le verra plus loin, est prolongé en sortie de la partie avant du corps de liaison 11, par un tube creux 15, flexible et avantageusement transparent. Dans le mode de réalisation illustré, le tube creux 15 s'étend dans l'axe de la seringue 1.

L'autre canal 14 définit un canal d'injection. Il présente une extrémité 14A débouchant dans le canal de guidage 13, avantageusement en amont du tube creux 15, et une extrémité 14B communiquant avec le débouché du réservoir 3 lorsque la tête d'injection 10 est en place sur la seringue 1.

La tête d'injection 10 comporte en outre une aiguille 16, avantageusement pleine, montée coulissante à l'intérieur du canal de guidage 13 pour passer d'une position dans laquelle l'extrémité distale 16A de l'aiguille 16, laquelle est préférablement pointue, dépasse de l'extrémité distale 15A du tube creux 15 (figure 1), de sorte à former un ensemble pénétrant, à une position dans laquelle l'extrémité distale 16A de l'aiguille 16 est positionnée en amont de l'extrémité du canal d'injection débouchant dans le canal de guidage 13 (figure 2), et inversement.

La position dans laquelle l'extrémité distale 16A de l'aiguille 16 dépasse de l'extrémité distale 15A du tube creux 15 définit ainsi une position dite de piquage, tandis que la position dans laquelle l'extrémité distale 16A de l'aiguille 16 est positionnée en amont de l'extrémité débouchante du canal d'injection 14 définit une position permettant de contrôler et de caractériser la présence d'un liquide 112 en provenance du site d'injection 110 et de caractériser ledit liquide 112 via, comme on le verra plus loin, le tube creux 15.

L'aiguille 16 est reliée solidairement à une pièce mobile par rapport au corps de liaison 11. Plus particulièrement cette pièce, par la suite nommée navette 18, est montée mobile en translation sur un bras 17 s'étendant en partie arrière du corps de liaison, parallèlement au réservoir 3, dans la direction opposée à la direction d'injection du fluide de comblement. Avantageusement, le bras 17 est couplé avec le corps de liaison 11 de sorte qu'un léger déplacement en translation du bras 17 par rapport au corps de liaison 11 suivant un axe parallèle à l'axe de la seringue est autorisé. De par cet arrangement, l'aiguille 16 est placée en position de piquage ou en position de contrôle et de caractérisation par déplacement de la navette 18 sur le bras 17, cette dernière entraînant l'aiguille 16 lors de son déplacement.

La navette 18 et le corps de liaison 11 comportent des moyens d'association réciproques permettant de maintenir la navette 18 au voisinage du corps de liaison 11 et ainsi l'aiguille 16 dans sa position de piquage. Dans le mode de réalisation illustré, la navette 18 est maintenue au corps de liaison 11 par encliquetage. Plus particulièrement, le corps de liaison 11 comporte en partie arrière un doigt 19 pourvu d'un ergot de retenu 20 arrangé pour venir s'enficher dans une rainure en forme de T prévue sur la navette 18, ouverte en direction du corps de liaison, ledit ergot 20 venant en appui avec les portions de retour de la forme en T de la rainure. La rainure ménagée dans la navette 18 et le doigt 19 du corps de liaison 11 constituent les moyens d'association réciproques. L'ergot de retenu 20 est mis en prise avec la rainure par l'intermédiaire d'un ergot 21 ménagé sur le bras 17 et arrangé pour former une rampe s'étendant dans l'espace 22 situé entre l'ergot de retenu 20 et le corps de liaison 11. Le bras 17 permet, lorsqu'il est actionné, de désengager la navette 18 de l'ergot de retenu 20 du corps de liaison 11. Le bras 17 sert à la fois de moyen de guidage pour la navette 18 et de moyen d'actionnement du déplacement de l'aiguille 16 par rapport au corps de liaison 11. Il est désigné par la suite de bras d'actionnement 17.

La tête d'injection 10 comporte des moyens de rappel élastique 23 de la navette 18 de préférence dans la position dans laquelle l'aiguille 16 est en position de contrôle et de caractérisation, ceci afin de protéger le manipulateur de la seringue 1 de toute sortie intempestive de l'aiguille 16 et donc éviter tout risque de blessure pour la personne manipulant la seringue 1 ou pour la personne destinée à recevoir l'injection. Dans le mode de réalisation illustré, les moyens de rappel sont formés par un ressort présentant une extrémité reliée au niveau de l'extrémité libre du bras d'actionnement 17, l'autre extrémité étant reliée à la navette 18.

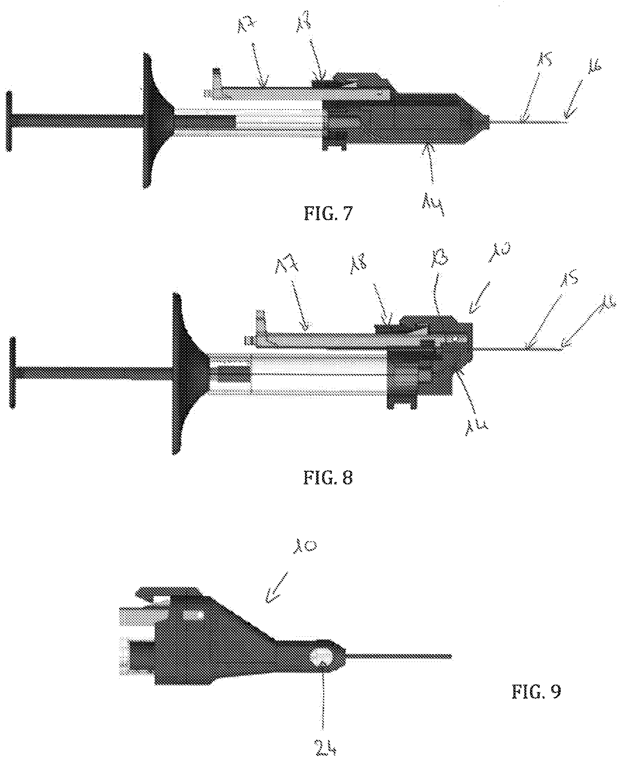

La tête d'injection 10 comporte en outre une zone de contrôle et de caractérisation du liquide 112 en provenance du site d'injection 110. Dans le mode de réalisation illustré, la zone de contrôle est constituée par le tube creux 15 lui-même, ce dernier étant avantageusement transparent ou localement transparent. Il peut être prévu cependant que la zone de contrôle et de caractérisation soit ménagée au niveau du corps de liaison 11 lui-même. La figure 9 représente un exemple de réalisation dans lequel le corps de liaison 11 comporte une fenêtre 24 de contrôle et de caractérisation du liquide.

Comme indiqué, l'aiguille 16 est une aiguille préférablement pleine (i.e. non creuse). Cet arrangement de l'aiguille 16 a pour fonction de favoriser le phénomène d'aspiration lors du passage de sa position de piquage à sa position de contrôle et de caractérisation, et ainsi assurer l'aspiration du liquide 112 éventuellement présente dans le site d'injection 110. Bien que la logique amène à utiliser une aiguille pleine, une aiguille creuse mais dont le canal a été obturé en aval de préférence, offre néanmoins un fonctionnement intéressant notamment de par sa souplesse mécanique.

Les figures 3a à 6b montrent la mise en oeuvre de la seringue venant d'être décrite pour l'injection du fluide de comblement 100 dans le site d'injection 110.

La première étape, illustrée sur la figure 3a consiste à armer la navette 18, c'est-à-dire à la mettre en prise avec le corps de liaison 11 via l'ergot de retenu 19, si elle ne l'est pas déjà, pour mettre l'aiguille 16 en position de piquage puis à piquer la seringue dans le site d'injection 110 dans lequel on souhaite injecter le fluide de comblement 100 (figure 3a, 3b).

On vérifie ensuite que l'ensemble pénétrant aiguille / tube creux n'est pas planté dans un vaisseau 111. Pour ce faire, le bras d'actionnement 17 est actionné en traction, avec un léger mouvement descendant (en retrait) en direction de la seringue, afin de libérer la navette 18 de l'ergot de retenu 19. La navette 18 libérée est entraînée par le ressort de rappel en direction de l'extrémité libre du bras d'actionnement 17, entraînant dans sa course l'aiguille 16 de sorte que l'extrémité 16A de l'aiguille 16 se retire du site d'injection 110 pour se positionner dans le canal de guidage, en amont de l'intersection du canal de guidage avec le canal d'injection (position de contrôle et de caractérisation de l'aiguille 16). Lors du retrait de l'extrémité 16A de l'aiguille 16 du site d'injection 110, une dépression se crée de sorte que si l'ensemble pénétrant est planté dans un vaisseau sanguin, du sang en provenance du vaisseau sanguin est entrainé à l'intérieur du tube creux 15 sous l'action de l'aspiration mais également de la pression artérielle. Ainsi, si le manipulateur voit apparaître du sang dans le tube creux 15 suite au passage de l'aiguille 16 de sa position de piquage à sa position de contrôle et de caractérisation, il sait que la seringue est plantée au niveau d'un vaisseau sanguin et dans ce cas qu'il ne doit pas injecter le fluide de comblement. Dans ce cas, il retirera la seringue et recommencera l'opération de piquage à un autre emplacement et procèdera à une nouvelle vérification. Si au contraire il ne voit pas remonter de sang à l'intérieur du tube creux 15, il procède à l'injection du produit de comblement par actionnement du piston 4 de la seringue comme illustrée sur les figures 5a et 5b (actionnement selon la flèche illustré sur la figure 5a), le produit de comblement étant entrainé depuis le réservoir vers le site d'injection 110 via le canal d'injection. Afin de procéder à l'injection complète du produit de comblement, il peut être prévu de remettre en prise la navette 18 avec le corps de liaison comme illustré sur les figures 6a et 6b. En remettant en prise la navette 18 avec le corps de liaison, l'aiguille 16 qui est pleine, entraine par poussée le produit de comblement présent, en aval de l'intersection entre le canal de guidage et le canal d'injection (et dans le tube creux »), dans le site d'injection 110.

Dans l'exemple venant d'être décrit, le piquage en sortie de la tête d'injection 10 est prévu dans l'axe de la seringue, le canal de guidage et le canal d'injection étant alors courbes et l'aiguille 16 forcée en courbe. D'autres arrangements peuvent être également prévus comme ceux par exemple illustrés sur les figures 7 et 8. Il peut être ainsi prévu un piquage en sortie de la tête d'injection 10 dans l'axe de la seringue mais avec un canal de guidage également axé avec la seringue de sorte que l'aiguille 16 mise en oeuvre est rectiligne. Seul le canal d'injection serait déporté en courbe (figure 7). Il peut être prévu également un piquage en sortie de la tête d'injection 10 désaxé par rapport à la seringue, avec un canal de guidage également axé sur l'axe de piquage (et une aiguille 16 associée rectiligne) et un canal d'injection en courbe (figure 8).

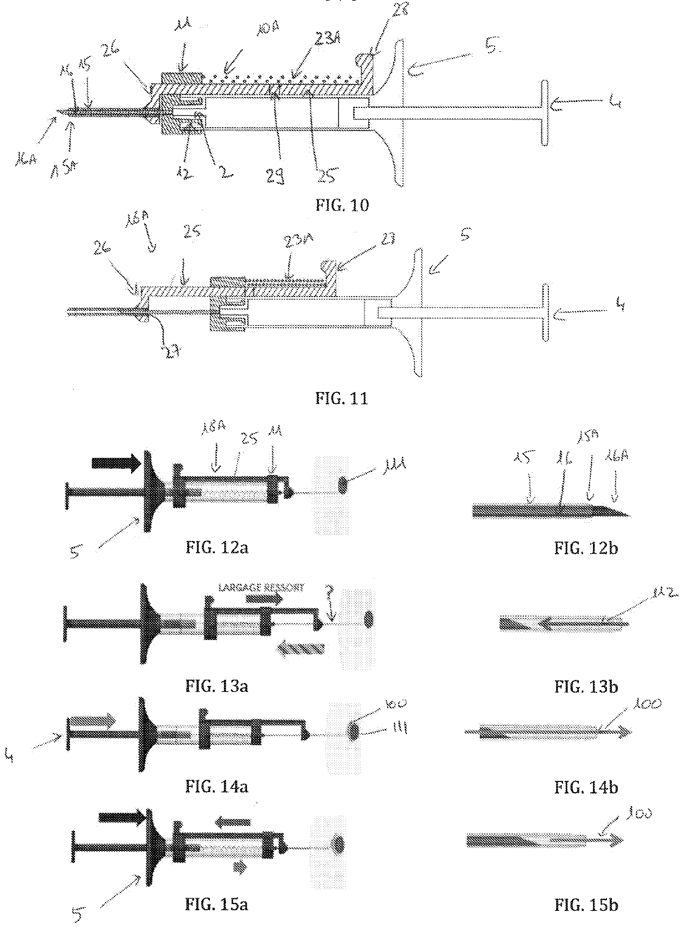

Les figures 10 et 11 illustrent une seringue pourvue d'une tête d'injection 10A selon un autre mode de réalisation ne faisant pas partie de l'invention.

Comme précédemment, la tête d'injection 10A comporte un corps de liaison 11 fixé sur la partie avant de la seringue 1 comprenant, en partie arrière, un élément de couplage 12 constituant une connexion du type Luer Lock femelle apte à coopérer avec l'embout 2 de la seringue 1, l'embout 2 formant une connexion du type Luer Lock mâle.

Le corps de liaison 11 est traversé par un canal d'injection prolongé en sortie de la partie avant du corps de liaison 11, par l'aiguille 16 et communiquant en entrée avec le débouché du réservoir 3 lorsque la tête d'injection 10A est en place sur la seringue 1.

Dans ce mode de réalisation, l'aiguille 16, qui est creuse, est solidaire du corps de liaison 11. Plus particulièrement, l'aguille est montée au travers du corps de liaison 11 et communique avec l'embout 2 de la seringue 1 lorsque la tête d'injection 10A est en place sur la seringue 1.

Le tube creux 15 quant à lui est solidaire de la navette 18A. Dans le mode de réalisation illustrée, la navette 18A comporte un bras 25 monté coulissant dans une rainure ménagée en partie supérieure du corps de liaison 11. Le bras 25 présente, en extrémité distale, une partie coudée 26 pourvue d'un orifice 27 au travers duquel l'aiguille 16 s'étend, le tube creux 15 étant relié solidairement à la partie coudée et s'étendant coaxialement à l'aiguille 16. Le coulissement de la navette 18A est actionné manuellement par l'intermédiaire d'une clavette d'actionnement 28 ménagée au niveau de l'extrémité proximale du bras 25.

Comme précédemment, la tête d'injection 10A comporte des moyens de rappel élastique 19 de la navette 18A (ou corps 11 de liaison) dans la position dans laquelle l'aiguille 16 est en position de contrôle et de caractérisation (figure 11), ceci afin de protéger le manipulateur de la seringue 1 de toute sortie intempestive de l'aiguille 16 et donc éviter tout risque de blessure pour la personne manipulant la seringue 1 ou pour la personne destinée à recevoir l'injection. Dans le mode de réalisation illustré, les moyens de rappel sont formés par un ressort 23A présentant une extrémité reliée au niveau de l'extrémité proximale du bras 25 de la navette 18A, l'autre extrémité étant reliée au corps de liaison 11.

Avantageusement, la portion de bras 25 s'étendant en amont du corps de liaison est pourvue d'ergots latéraux 29 pour assurer l'arrêt de la navette 18A en position de contrôle et de caractérisation lorsque la clavette d'actionnement 28 est relâchée et la navette 18A entrainée par le ressort pour passer de la position de piquage à la position de contrôle et de caractérisation illustrée sur la figure 11.

Les figures 12a à 15b montrent la mise en oeuvre de la seringue venant d'être décrite pour l'injection du fluide de comblement 100 dans le site d'injection 110 visé.

La première étape, illustrée sur la figure 12a consiste à placer le bras 25 de la navette 18A en arrière en plaçant le pouce sur la face arrière de la garde 5 de la seringue et l'index sur la face avant de la clavette d'actionnement 28 et en tirant cette dernière vers l'arrière, en direction de la garde 5 de sorte à mettre l'aiguille 16 en position de piquage (extrémité distale de l'aiguille 16 dépassant de l'extrémité distale du tube creux 15 comme représenté sur la figure 12b), à maintenir le bras 25 tiré vers l'arrière et à planter la seringue dans le site d'injection 110 dans lequel on souhaite injecter le fluide de comblement 100.

On vérifie ensuite que l'ensemble pénétrant n'est pas planté dans un vaisseau sanguin 111. Pour ce faire, la pression exercée sur la garde 5 est relâchée tout en maintenant la position de la navette 18A par rapport au site d'injection 110. La seringue ainsi libérée, et le corps de liaison portant l'aiguille 16 solidaire de la seringue, sont déplacés en translation vers l'arrière, en direction opposée au site d'injection 110, par rapport à la navette 18A, entraînant dans leur déplacement l'aiguille 16 laquelle se retire du site d'injection 110 pour se positionner en retrait de l'extrémité distale du tube creux 15, en position de contrôle et de caractérisation (figures 13a et 13b). Comme précédemment, la dépression est créée par le retrait de l'aiguille 16 ou la simple pression sanguine, de sorte que si l'ensemble pénétrant est planté dans un vaisseau sanguin, du sang en provenance du vaisseau sanguin est entrainé à l'intérieur du tube creux 15 sous l'action de l'aspiration et/ou de la pression artérielle. Ainsi, si le manipulateur voit apparaître du sang dans le tube creux 15 lors du passage de l'aiguille 16 de sa position de piquage à sa position de contrôle et de caractérisation, il sait que la seringue est plantée au niveau d'un vaisseau sanguin et dans ce cas qu'il ne doit pas injecter le fluide de comblement. Dans ce cas, il retirera la seringue et recommencera l' opération de piquage à un autre emplacement et procèdera à une nouvelle vérification. Si au contraire il ne voit pas remonter de sang à l'intérieur du tube creux 15, il procède à l'injection du produit de comblement par actionnement du piston de la seringue comme illustré sur les figures 14a et 14b (actionnement selon la flèche illustré sur la figure 14a), le produit de comblement étant entrainé depuis le réservoir vers le site d'injection 110 via l'aiguille 16 et le tube creux 15. Afin de procéder à l'injection complète du produit de comblement pour évacuer la substance présente et restante dans le tube creux, vers le site d'injection (étape facultative), il peut être prévu de replacer le bras 25 de la navette 18A en arrière en actionnant, conjointement la garde et la clavette d'actionnement 28 tout en maintenant le tube creux implanté dans le site d'injection 110 (figures 15a et 15b) ou avoir la possibilité de le réimplanter ailleurs.

Dans le cas du premier mode de réalisation illustré sur les figures 1 à 6B, l'aiguille est pleine de sorte que le déplacement de cette dernière dans le tube creux en direction opposée au site d'injection crée une dépression appelant le liquide. Dans le cas du deuxième mode de réalisation illustré sur les figures 10 à 15B, l'aiguille est creuse mais débouche dans la seringue remplit de fluide à injecter. Du fait de la faible fluidité du fluide d'injection, la translation de l'aiguille dans le tube crée aussi une dépression dans le tube. Une telle dépression est avantageuse pour l'appel de sang mais n'est pas strictement nécessaire. Ainsi par exemple, s'il y a une fuite d'air entre le tube creux et l'aiguille, le sang remontera quand même jusqu'à la zone de contrôle et de caractérisation de par sa fluidité et la relative pression dans laquelle il se trouve dans le corps, tandis que ladite fuite d'air n'aura pas de conséquence sur la dynamique d'injection de par la différence de viscosité du fluide et de l'air. Une autre conception peut aussi consister en une zone de contrôle et de caractérisation qui soit simplement un trou débouchant situé en partie proximale du tube creux ou en amont du tube creux notamment le long du canal de guidage dans sa section dégagée de l'aiguille pleine en position de contrôle et de caractérisation. En effet, s'agissant d'une situation d'alerte exceptionnelle, le fait que le sang coule ainsi dans ce cas particulier peut être acceptable dans certains usages. On notera aussi qu'un trou de faible diamètre, par exemple de 0.05 mm ou 0.1 mm peut être apte à faire passer la pression atmosphérique et le sang, mais ne pas laisser fuir le fluide de comblement, qui est le plus souvent un produit de comblement très visqueux.

Les modes de réalisation précédemment décrits se rapportent à une tête d'injection 10 d'une substance fluide exogène. Il est bien entendu évident que l'invention ne se limite pas à ce type de substances fluidiques, la tête d'injection selon l'invention pouvant être mise en oeuvre pour l'injection également de substances endogènes préalablement extraites d'un corps sans sortir du cadre de l'invention.

Dans les exemples illustrés, la tête d'injection est associée à une seringue ou un corps de seringue. Elle peut être également mise en oeuvre avec un système de délivrance de type pousse-seringue ou pompe qui injecte la substance fluide via un tuyau jusqu'à la tête d'injection sans sortir du cadre de l'invention.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Tête d'injection (10) d'une substance fluide (100) destinée à être injectée dans un site d'injection (110) corporel en passant la barrière cutanée, ladite tête permettant, préalablement à l'injection, le contrôle de la présence d'un liquide (112) en provenance du site d'injection (110) et la caractérisation dudit liquide, la tête d'injection comportant un corps de liaison (11) destiné à être connecté à un réservoir dont la pression peut être contrôlée, ledit réservoir étant destiné à contenir la substance fluide (100) à injecter, ledit corps de liaison (11) étant prolongé par un tube creux (15), destiné à communiquer avec le débouché du réservoir, présentant une extrémité distale et une aiguille (16) montée coulissante dans un canal de guidage traversant le corps de liaison (11), ladite aiguille présentant une extrémité distale pointue, ledit tube creux (15) ou ledit corps de liaison (11) présentant au moins une zone de contrôle et de caractérisation du liquide (112) en provenance du site d'injection (110),
**caractérisé en ce que** le tube creux (15) communique avec le débouché du réservoir via un canal d'injection traversant le corps de liaison (11), ledit canal d'injection présentant une extrémité débouchante (14A) dans le canal de guidage et une extrémité (14B) communiquant avec le débouché du réservoir lorsque la tête d'injection (10) est connectée au réservoir, et
**en ce que** la tête d'injection comporte des moyens pour commander le déplacement relatif de ladite aiguille (16) par rapport audit tube creux (15), entre une position de piquage dans laquelle l'extrémité pointue de l'aiguille (16), laquelle est gigogne dans le tube creux (15), dépasse l'extrémité distale du tube creux (15), pour former, avec ladite extrémité distale, un ensemble pénétrant, et une position de contrôle et de caractérisation du fluide (112) en provenance du site d'injection (110) via la zone de contrôle et de caractérisation dans laquelle l'extrémité distale pointue de l'aiguille (16) étant positionnée dans le canal de guidage, en amont de l'extrémité du canal d'injection débouchant dans le canal de guidage (13), et inversement, lesdits moyens de commande comportant une navette (18) montée mobile par rapport au corps de liaison (11) pour placer l'aiguille (16) en position de piquage ou en position de contrôle et de caractérisation d'un liquide (112) en provenance du site d'injection (110), l'aiguille (16) étant solidaire de la navette (18) tandis que le tube creux (15) est solidaire du corps de liaison (11) et des moyens de rappel élastique de la navette (18) pour placer l'aiguille (16) en position de piquage ou en position de contrôle et de caractérisation d'un liquide (112) en provenance du site d'injection (110).

2. Tête d'injection (10) selon la revendication 1, **caractérisée en ce que** le corps de liaison (11) comporte des moyens de guidage coopérant avec des moyens de guidage complémentaires ménagés sur la navette (18) de sorte à guider la navette (18) dans son parcours.

3. Tête d'injection (10) selon la revendication 1 ou la revendication 2, **caractérisée en ce que** la navette (18) et le corps de liaison (11) comportent des moyens de maintien coopérant de sorte à maintenir l'aiguille (16) dans sa position de piquage ou sa position de contrôle et de caractérisation du liquide (112) en provenance du site d'injection (110).

4. Tête d'injection (10) selon l'une quelconque des revendications 1 à 3, **caractérisée en ce que** les moyens de commande sont arrangés pour commander le déplacement de l'aiguille (16) dans le canal de guidage dans une position d'injection de la substance liquide dans laquelle l'aiguille (16) est positionnée en amont de l'extrémité débouchante du canal d'injection.

5. Tête d'injection (10) selon l'une quelconque des revendications 1 à 4, **caractérisée en ce que** l'aiguille (16) est pleine.

6. Seringue (1) équipée, en partie avant, d'une tête d'injection (10) selon l'une quelconque des revendications précédentes, ladite tête d'injection (10) étant raccordée au réservoir (3) de la seringue (1).

7. Seringue selon la revendication 6, **caractérisée en ce que** la tête d'injection (10) et le réservoir de la seringue (1) constituent deux pièces indépendantes fixables l'une à l'autre.

8. Seringue selon la revendication 6 ou la revendication 7, **caractérisée en ce que** la tête d'injection (10) est amovible.

9. Seringue selon l'une quelconque des revendications 6 à 8, **caractérisé en ce que** le réservoir (3) est pré-rempli.

## Patentansprüche

1. Injektionskopf (10) für eine fluidische Substanz (100), die dazu bestimmt ist, in eine Körperinjektionsstelle (110) injiziert zu werden, indem sie die Hautbarriere passiert, wobei der Kopf vor der Injektion die Überprüfung des Vorhandenseins einer Flüssigkeit (112) aus der Injektionsstelle (110) und die Kennzeichnung der Flüssigkeit ermöglicht, wobei der Injektionskopf einen Verbindungskörper (11) umfasst, der dazu bestimmt ist, mit einem Behälter verbunden zu werden, dessen Druck überprüft werden kann, wobei der Behälter dazu bestimmt ist, die zu injizierende fluidische Substanz (100) zu enthalten, der Verbindungskörper (11) durch ein Hohlrohr (15) verlängert ist, das dazu bestimmt ist, mit der Mündung des Behälters zu kommunizieren, das ein distales Ende und eine Hohlnadel (16) aufweist, die in einem den Verbindungskörper (11) durchquerenden Führungskanal gleitend montiert ist, wobei die Hohlnadel ein spitzes distales Ende aufweist, wobei das Hohlrohr (15) oder der Verbindungskörper (11) mindestens eine Zone zur Überprüfung und Kennzeichnung der Flüssigkeit (112) von der Injektionsstelle (110) aufweist,
**dadurch gekennzeichnet, dass** das Hohlrohr (15) über einen den Verbindungskörper (11) durchquerenden Injektionskanal mit der Mündung des Behälters in Verbindung steht, wobei der Injektionskanal ein in den Führungskanal mündendes Ende (14A) und ein mit der Mündung des Behälters in Verbindung stehendes Ende (14B), wenn der Injektionskopf (10) mit dem Behälter verbunden ist, aufweist und
dass der Injektionskopf Mittel zum Steuern der relativen Bewegung der Hohlnadel (16) in Bezug auf das Hohlrohr (15) zwischen einer Einstichposition, in der das spitze Ende der Hohlnadel (16), die in das Hohlrohr (15) einschiebbar ist, das distale Ende des Hohlrohrs (15) überragt, um mit dem distalen Ende eine durchdringende Anordnung auszubilden, und einer Überprüfungs- und Kennzeichnungsposition für das Fluid (112) von der Injektionsstelle (110) über den Überprüfungs- und Kennzeichnungsbereich, umfasst, wobei das spitze distale Ende der Hohlnadel (16) in dem Führungskanal stromaufwärts des Endes des Injektionskanals, das in den Führungskanal (13) mündet, und umgekehrt, positioniert ist, wobei die Steuermittel ein Schiffchen (18) umfassen, das in Bezug auf den Verbindungskörper (11) bewegbar montiert ist, um die Hohlnadel (16) in die Einstechposition oder in die Position zur Überprüfung und Kennzeichnung einer Flüssigkeit (112) von der Injektionsstelle (110) zu bringen, wobei die Hohlnadel (16) mit dem Schiffchen (18) fest verbunden ist, während das Hohlrohr (15) mit dem Verbindungskörper (11) und den elastischen Rückstellmitteln des Schiffchens (18) fest verbunden ist, um die Hohlnadel (16) in die Einstichposition oder in die Position zur Überprüfung und Kennzeichnung einer Flüssigkeit (112) von der Injektionsstelle (110) zu platzieren.

2. Injektionskopf (10) nach Anspruch 1, **dadurch gekennzeichnet, dass** der Verbindungskörper (11) Führungsmittel umfasst, die mit komplementären Führungsmitteln zusammenwirken, die auf dem Schiffchen (18) vorgesehen sind, um das Schiffchen (18) auf seinem Weg zu führen.

3. Injektionskopf (10) nach Anspruch 1 oder 2,
**dadurch gekennzeichnet, dass** das Schiffchen (18) und der Verbindungskörper (11) Haltemittel umfassen, die zusammenwirken, um die Hohlnadel (16) in ihrer Einstichposition oder ihrer Position zur Überprüfung und Kennzeichnung der Flüssigkeit (112) von der Injektionsstelle (110) zu halten.

4. Injektionskopf (10) nach einem der Ansprüche 1 bis 3,
**dadurch gekennzeichnet, dass** die Steuermittel eingerichtet sind, um die Bewegung der Hohlnadel (16) in dem Führungskanal in einer Position zur Injektion der flüssigen Substanz zu steuern, in der die Hohlnadel (16) stromaufwärts des Endes, das in den Injektionskanal mündet, positioniert ist.

5. Injektionskopf (10) nach einem der Ansprüche 1 bis 4,
**dadurch gekennzeichnet, dass** die Hohlnadel (16) gefüllt ist.

6. Spritze (1), die im vorderen Teil mit einem Injektionskopf (10) nach einem der vorstehenden Ansprüche ausgestattet ist, wobei der Injektionskopf (10) an den Behälter (3) der Spritze (1) angeschlossen ist.

7. Spritze nach Anspruch 6, **dadurch gekennzeichnet, dass** der Injektionskopf (10) und der Behälter der Spritze (1) zwei unabhängige, aneinander befestigbare Teile bilden.

8. Spritze nach Anspruch 6 oder 7, **dadurch gekennzeichnet, dass** der Injektionskopf (10) abnehmbar ist.

9. Spritze nach einem der Ansprüche 6 bis 8,
**dadurch gekennzeichnet, dass** der Behälter (3) vorgefüllt ist.

## Claims

1. Injection head (10) for a fluid substance (100) intended to be injected into an injection site (110) into the body by passing through the skin barrier, said head allowing, prior to the injection, the monitor of the presence of a liquid (112) originating from the injection site (110) and the characterization of said liquid, the injection head comprising a connection body (11) intended to be connected to a reservoir, the pressure of which can be controlled, said reservoir being intended to contain the fluid substance (100) to be injected, said connection body (11) being extended by a hollow tube (15) intended to communicate with the reservoir outlet, the tube having a distal end and a needle (16) that is slidably mounted in a guide channel passing through the connection body (11), said needle having a pointed distal end, said hollow tube (15) or said connection body (11) having at least one zone for monitoring and characterizing the liquid (112) coming from the injection site (110),
**characterized in that** the hollow tube (15) communicates with the reservoir outlet via an injection channel passing through the connection body (11), said injection channel having an end (14A) emerging into the guide channel and an end (14B) communicating with the reservoir outlet when the injection head (10) is connected to the reservoir, and
**in that** the injection head comprises means for controlling the relative movement of said needle (16) in relation to said hollow tube (15), between a pricking position in which the pointed end of the needle (16), which is nested within the hollow tube (15), projects beyond the distal end of the hollow tube (15) in order to form, with said distal end, a penetrating assembly, and a position for monitoring and characterizing the fluid (112) originating from the injection site (110) via the monitoring and characterization zone, in which the pointed distal end of the needle (16) is positioned in the guide channel, upstream of the end of the injection channel which emerges into the guide channel (13), and vice versa, said control means comprising a shuttle (18) movably mounted relative to the connection body (11) in order to place the needle (16) in the pricking position or in the position for monitoring and characterizing a liquid (112) coming from the injection site (110), the needle (16) being secured to the shuttle (18) whereas the hollow tube (15) is secured to the connection body (11), and resilient return means for the shuttle (18) for placing the needle (16) in the pricking position or in the position for monitoring and characterizing a liquid (112) originating from the injection site (110).

2. Injection head (10) according to claim 1, **characterized in that** the connection body (11) comprises guiding means which engage with complementary guiding means provided on the shuttle (18) so as to guide the shuttle (18) in its path.

3. Injection head (10) according to claim 1 or claim 2, **characterized in that** the shuttle (18) and the connection body (11) comprise holding means which engage to hold the needle (16) in the pricking position or the position for monitoring and characterizing the liquid (112) coming from the injection site (110).

4. Injection head (10) according to any one of claims 1 to 3,
**characterized in that** the control means are arranged to control the movement of the needle (16) in the guide channel into a position for injecting the liquid substance in which the needle (16) is positioned upstream of the emerging end of the injection channel.

5. Injection head (10) according to any one of claims 1 to 4,
**characterized in that** the needle (16) is solid.

6. Syringe (1) equipped, at the front, with an injection head (10) according to any one of the preceding claims, said injection head (10) being connected to the reservoir (3) of the syringe (1).

7. Syringe according to claim 6, **characterized in that** the injection head (10) and the reservoir of the syringe (1) constitute two independent parts which are attachable to one another.

8. Syringe according to claim 6 or claim 7, **characterized in that** the injection head (10) is removable.

9. Syringe according to any one of claims 6 to 8, **characterized in that** the reservoir (3) is pre-filled.
